# EUROPÄISCHE PATENTANMELDUNG

(11) **EP 3 120 836 A1**
(43) Veröffentlichungstag der Anmeldung: **25.01.2017**
(21) Anmeldenummer: 15002163.2
(22) Anmeldetag: 22.07.2015
(51) Int. Cl.: A61K 9/08, A61K 31/69

(54) **GEBRAUCHSFERTIGE BORTEZOMIB-LÖSUNG**

(71) Anmelder: STADA ARZNEIMITTEL AG, 61118 Bad Vilbel (DE)
(72) Erfinder: SCHRIDDE, Edgar, 30855 Langenhagen (DE)
(74) Vertreter: Kernebeck, Thomas

(57) **Zusammenfassung**

Die vorliegende Erfindung betrifft eine gebrauchsfertige flüssige pharmazeutische Bortezomib-Lösung. Um eine Bortezomib-Lösung der gattungsgemäßen Art bereitzustellen, die eine verbesserte physiologische Toleranz aufweist, wird eine gebrauchsfertige flüssige pharmazeutische Lösung vorgeschlagen, umfassend
- ein wässriges Lösungsmittel;
- einen Bortezomibester, der in dem Lösungsmittel gelöst enthalten ist.

## Beschreibung

Die vorliegende Erfindung betrifft eine gebrauchsfertige flüssige pharmazeutische Bortezomib-Lösung.

Bortezomib ist der internationale Freiname (INN (*international nonproprietary name*)) für (1R)-3-Methyl-1-[((2S)-3-phenyl-2-[(2-pyrazinylcarbonyl)-amino]propanoyl)amino]butylborsäure, die die folgende Strukturformel aufweist:

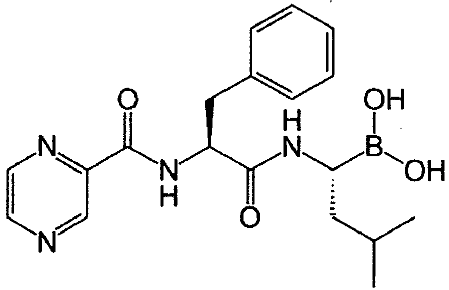

Bortezomib ist ein Arzneistoff aus der Gruppe der Proteasom-Inhibitoren, der für die Mono- und Kombinationstherapie zur Behandlung des multiplen Myeloms und des Mantelzelllymphoms zugelassen ist. Die Wirkung von Bortezomib beruht dabei auf der selektiven Hemmung der Chymotrypsin-artigen Aktivität des 26S-Proteasoms (vgl. Mutschler Arzneimittelwirkungen, Lehrbuch der Pharmakologie und Toxikologie, 9. Auflage, Wissenschaftliche Verlagsgesellschaft mbH Stuttgart, 2008, S. 947; ISBN 978-3-8047-1952-1).

Bortezomib wird unter der Markenbezeichnung Velcade^{®} in Form eines lyophilisierten Pulvers in den Wirkstärken 1 mg und 3,5 mg Bortezomib vertrieben. Bortezomib ist in Velcade^{®} in Form eines Mannitol-Boronsäureesters enthalten, der nach Rekonstitution die pharmazeutisch aktive Komponente Bortezomib *in vivo* relativ gut freisetzen soll. Für die intravenöse Verabreichung wird das in einer Durchstechflasche (Vial) enthaltene sterile Pulver mit einer 0,9 %igen Kochsalzlösung rekonstituiert, wobei eine Lösung mit 1 mg/ml Bortezomib zur intravenösen oder 2,5 mg/ml Bortezomib zur subkutanen Applikation hergestellt wird. Die Lagerstabilität einer ungeöffneten Durchstechflasche Velcade^{®} wird bei einer Temperatur bis zu 30 °C mit 3 Jahren angegeben, die einer durch Rekonstitution von Velcade^{®} erhaltenen Bortezomib-Lösung bei 25 °C hingegen nur mit 8 Stunden.

Im Hinblick auf eine sicherere Handhabung des Zytostatikums Bortezomib wäre es wünschenswert, wenn man Bortezomib in Form einer gebrauchsfertigen Lösung am Markt anbieten könnte. Zwar ist Bortezomib-Mannitolester in fester Formulierung verhältnismäßig stabil, nicht jedoch in wässriger Formulierung, da freies Bortezomib in wässriger Lösung sehr oxidationsempfindlich ist und Bortezomib-Mannitolester in Lösung im Gleichgewicht mit seinen Hydrolyseprodukten freies Bortezomib und Mannitol vorliegt.

Entsprechend schlägt WO 2011/116286 A2 eine gebrauchsfertige Bortezomib-Lösung auf Basis des organischen Lösungsmittels Propylenglykol vor. Es wird behauptet, dass eine derartige Lösung die an eine gebrauchsfertige Lösung zu stellenden Anforderungen hinsichtlich Stabilität und damit Lagerfähigkeit erfüllt. Nachteilig an besagter Lösung ist allerdings, dass Propylenglykol starke Reizungen verursachen sowie zu Leberanomalien und Nierenschäden führen kann.

Aufgabe der vorliegenden Erfindung ist es daher, eine gebrauchsfertige flüssige pharmazeutische Bortezomib-Lösung bereitzustellen, die eine verbesserte physiologische Toleranz aufweist.

Diese Aufgabe wird gelöst durch eine gebrauchsfertige flüssige pharmazeutische Lösung, umfassend
- ein wässriges Lösungsmittel;
- einen Bortezomibester, der in dem Lösungsmittel gelöst enthalten ist.

Überraschenderweise wurde festgestellt, dass eine gebrauchsfertige flüssige pharmazeutische Bortezomib-Lösung, enthaltend Bortezomib in Form eines Esters, auf der Basis eines wässrigen Lösungsmittels bereitgestellt werden kann. Die erfindungsgemäße Lösung zeichnet sich durch eine verbesserte physiologische Toleranz aus.

Die erfindungsgemäße Lösung weist ferner den Vorteil auf, dass sie stabil ist.

Unter dem Begriff "Stabilität" wird in diesem Zusammenhang die von der Arbeitsgemeinschaft für pharmazeutische Verfahrenstechnik (APV) erarbeitete Definition verstanden, nach welcher "Stabilität" die spezifikationsgerechte Qualität des Arzneimittels bis zum Ende der vom Hersteller festgelegten Laufzeit bedeutet. Die Qualität des Arzneimittels wird dabei durch den Wirkstoffgehalt und die Reinheit, die sensorisch wahrnehmbaren, physikalisch-chemischen und die mikrobiologischen Eigenschaften bestimmt, wobei der Wirkstoffgehalt bis zum Ende der Laufzeit 90 % des deklarierten Wertes nicht unterschreiten soll.

Die erfindungsgemäße Lösung weist bei einer Temperatur von 2 bis 8 °C eine Stabilität (oder anders gesagt Lagerfähigkeit oder Laufzeit) von zumindest 3 Monaten auf, bevorzugt von zumindest 6 Monaten, weiter bevorzugt von zumindest 12 Monaten, noch weiter bevorzugt von zumindest 24 Monaten und am meisten bevorzugt von zumindest 36 Monaten, wobei der Bortezomibgehalt bis zum Ende der Laufzeit 90 %, bevorzugt 95 %, des ursprünglich eingesetzten Bortezomibs nicht unterschreitet.

Die erfindungsgemäße Lösung weist ferner erfindungsgemäß bevorzugt bei einer Temperatur von 25 °C eine Stabilität (oder anders gesagt Lagerfähigkeit oder Laufzeit) von zumindest 3 Monaten auf, bevorzugt von zumindest 6 Monaten, weiter bevorzugt von zumindest 12 Monaten und noch weiter bevorzugt von zumindest 24 Monaten, wobei der Bortezomibgehalt bis zum Ende der Laufzeit 90 %, bevorzugt 95 %, des ursprünglich eingesetzten Bortezomibs nicht unterschreitet.

Die vorliegende Erfindung betrifft eine gebrauchsfertige flüssige pharmazeutische Wirkstoff-Lösung mit Bortezomib als Wirkstoff. Gebrauchsfertige Wirkstoff-haltige Lösungen sind im Stand der Technik bekannt und werden dort auch als *ready-to-use* (RTU)-Lösungen bezeichnet. Während als Lösung zu applizierende feste Wirkstoffformulierungen z.B. in Form von Pulvern oder Lyophilisaten in den Markt kommen und erst kurz vor ihrer Verabreichung mittels Rekonstitution in Lösung überführt werden, werden gebrauchsfertige Lösungen bereits in Form von Lösungen vermarktet.

Die erfindungsgemäße Lösung kann beispielsweise unmittelbar, d.h. ohne weitere Zubereitung verabreicht werden oder auch erst nach einer weiteren Zubereitung wie etwa einem Verdünnen auf eine gewünschte Wirkstoffkonzentration.

Die erfindungsgemäße gebrauchsfertige Lösung umfasst ein wässriges Lösungsmittel. Im Rahmen der vorliegenden Erfindung wird unter einem wässrigen Lösungsmittel ein Lösungsmittel bzw. ein Lösungsmittelgemisch von bei einer Temperatur von 20 °C und einem Druck von 1013 mbar flüssigen Einzelkomponenten verstanden, das zumindest zu 50 Gew.-% aus Wasser besteht, bevorzugt zumindest zu 70 Gew.-% aus Wasser, mehr bevorzugt zumindest zu 90 Gew.-% aus Wasser und noch mehr bevorzugt zumindest zu 95 Gew.-% aus Wasser. In diesem Zusammenhang ist es erfindungsgemäß besonders bevorzugt, wenn das Lösungsmittel zumindest zu 99 Gew.-% aus Wasser besteht und noch weiter bevorzugt zu 100 Gew.-% aus Wasser.

Neben Wasser kann das Lösungsmittel der erfindungsgemäßen Lösung ferner einen gewissen Anteil von einer oder mehreren mit Wasser mischbaren flüssigen organischen Komponenten enthalten. Als Beispiele für derartige organische Komponenten seien Ethanol und Isopropanol genannt.

In der erfindungsgemäßen Lösung ist ein Bortezomibester gelöst enthalten. Bortezomib ist eine sogenannte Boronsäure, die die funktionelle Gruppe -B(OH)₂ enthält, welche mit einer eine oder mehrere Alkohol-Gruppen enthaltenden Bortezomibester-Bildungskomponente unter Abspaltung von Wasser und gegebenenfalls Säure verestert werden kann. Erfindungsgemäß können sämtliche Bortezomibester von Bortezomib mit einer Bortezomibester-Bildungskomponente eingesetzt werden, die für den erfindungsgemäßen pharmazeutischen Zweck geeignet sind. Erfindungsgemäß bevorzugt ist es jedoch, wenn der Bortezomibester ein Ester des Bortezomibs mit einem Polyol als Bortezomibester-Bildungskomponente ist. Dadurch wird eine verhältnismäßig hohe Stabilität der erfindungsgemäßen gebrauchsfertigen Lösung gewährleistet. Erfindungsgemäß bevorzugt sind dabei insbesondere solche Bortezomib-Polyolester, in denen die beiden OH-Gruppen der Gruppe -B(OH)2 eines Bortezomib-Moleküls mit zwei OH-Gruppen eines Polyol-Moleküls unter Abspaltung von Wasser und Ausbildung einer Ringstruktur verestert sind, wie zum Beispiel in EP 1 355 910 anhand von Strukturformeln gezeigt.

Gemäß einer weiter bevorzugten Ausführungsform der erfindungsgemäßen Lösung ist es vorgesehen, dass die Bortezomibester-Bildungskomponente ein Alditol ist. Eine erfindungsgemäße Lösung enthaltend einen Bortezomib-Alditolester ist durch eine verhältnismäßig hohe Stabilität gekennzeichnet. Erfindungsgemäß bevorzugt sind dabei insbesondere solche Bortezomib-Alditolester, in denen die beiden OH-Gruppen der Gruppe -B(OH)2 eines Bortezomib-Moleküls mit zwei OH-Gruppen eines Alditol-Moleküls unter Abspaltung von Wasser und Ausbildung einer Ringstruktur verestert sind, wie zum Beispiel in EP 1 355 910 anhand von Strukturformeln gezeigt.

Alditole sind im Stand der Technik bekannt und entsprechen der nachstehenden allgemeinen Strukturformel, wobei n aus den natürlichen Zahlen ausgewählt und größer/gleich 1 ist

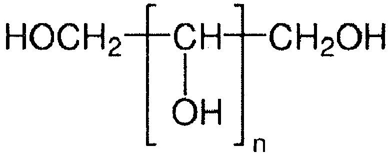

Entsprechend einer weiter bevorzugten Ausführungsform der erfindungsgemäßen Lösung ist es vorgesehen, dass die Bortezomibester-Bildungskomponente ein Alditol der voranstehenden Strukturformel mit n=1 bis 10 ist, vorzugsweise ein Alditol mit n=2 bis 4. Erfindungsgemäße Lösungen enthaltend einen Bortezomib-Alditolester besagter Auswahl zeichnen sich durch eine verhältnismäßig hohe Stabilität und hohe Löslichkeit aus.

Nach einer weiter bevorzugten Ausführungsform der erfindungsgemäßen Lösung ist es vorgesehen, dass die Bortezomibester-Bildungskomponente ausgewählt ist aus der Gruppe bestehend aus Arabitol, Dulcitol, Erythritol, Mannitol, Ribitol, Sorbitol und Xylitol und insbesondere aus der Gruppe bestehend aus Mannitol, Sorbitol und Xylitol, wobei Mannitol und dabei insbesondere D-Mannitol besonders bevorzugt ist. Erfindungsgemäße Lösungen enthaltend einen Bortezomib-Alditolester besagter Alditole zeichnen sich durch eine besonders hohe Stabilität und hohe Löslichkeit aus.

In der erfindungsgemäßen Lösung liegt der Bortezomibester im Gleichgewicht mit seinen Hydrolyseprodukten freies Bortezomib und freie Bortezomibester-Bildungskomponente vor. Um die Stabilität der erfindungsgemäßen Lösung weiter zu erhöhen, ist es entsprechend einer weiter bevorzugten Ausführungsform der Erfindung vorgesehen, dass die Bortezomibester-Bildungskomponente in einem zumindest 10-fachen molaren Überschuss in der Lösung gelöst enthalten ist in Bezug auf in der Lösung enthaltenem Bortezomib sowohl in freier als auch in veresterter Form, bevorzugt in einem 10 bis 400-fachen molaren Überschuss und besonders bevorzugt in einem 10 bis 100-fachen molaren Überschuss und ganz bevorzugt in einem 15 bis 30-fachen molaren Überschuss.

Gemäß einer weiter bevorzugten Ausführungsform der erfindungsgemäßen Lösung ist es vorgesehen, dass zumindest 90 % des in der Lösung enthaltenen Bortezomibs in Form eines Esters vorliegen, weiter bevorzugt zumindest 95 % und noch mehr bevorzugt zumindest 99 %. Dadurch wird eine verhältnismäßig hohe Stabilität der erfindungsgemäßen Lösung gewährleistet. Der Grad der Veresterung des Bortezomibs in der erfindungsgemäßen Lösung kann durch dem Fachmann bekannte Maßnahmen eingestellt werden, wie z.B. durch den pH-Wert der Lösung oder über den Gehalt der Lösung an freier Bortezomibester-Bildungskomponente. Der Grad der Veresterung des Bortezomibs wird erfindungsgemäß bevorzugt durch eine Titration und des ermittelten apparenten pka-Wertes des Bortezomibs oder durch ¹¹Bor-NMR bestimmt (siehe W. Marinaro, Physical and chemical properties of boronic acids: Formulation implications, Dissertation University Kansas, ProQuest, UMI Dissertations Publishing 2007, ISBN-13: 978-0-549-13739-9).

Entsprechend einer weiter bevorzugten Ausführungsform der erfindungsgemäßen Lösung ist es vorgesehen, dass der Gehalt der Lösung an Bortezomibester bezogen auf Bortezomib 0,5 mg/ml bis 10 mg/ml beträgt, bevorzugt 1,0 mg/ml bis 5 mg/ml und besonders bevorzugt 2,5 mg/ml.

Gemäß einer weiter bevorzugten Ausführungsform der erfindungsgemäßen Lösung ist es vorgesehen, dass in der Lösung ferner NaCl gelöst enthalten ist. Es konnte gezeigt werden das NaCl die Stabilität der erfindungsgemäßen Lösung weiter verbessert.

Nach einer weiter bevorzugten Ausführungsform der erfindungsgemäßen Lösung ist es vorgesehen, dass die Lösung bei einer Temperatur von 20 °C einen pH-Wert in einem Bereich von 3 bis 6 aufweist, bevorzugt in einem Bereich von 3 bis 5 und besonders bevorzugt in einem Bereich von 3,5 bis 4,5. Es konnte gezeigt werden, dass die erfindungsgemäße Lösung in den genannten pH-Wert-Bereichen durch eine besonders hohe Stabilität gekennzeichnet ist.

In einer weiter bevorzugten Ausführungsform ist es vorgesehen, zur Einstellung des pH-Werts der erfindungsgemäßen Lösung Salzsäure oder Natriumhydroxid zu verwenden, je nachdem welcher pH-Wert eingestellt werden soll.

Um den pH-Wert der erfindungsgemäßen Lösung in einem pH-Wert Bereich von 3,7 bis 5,7 möglichst konstant zu halten und damit einhergehend eine erhöhte Stabilität der erfindungsgemäßen Lösung dauerhaft zu gewährleisten, ist es entsprechend einer weiter bevorzugten Ausführungsform der erfindungsgemäßen Lösung vorgesehen, dass in der Lösung ein Essigsäure/Acetat-Puffersystem gelöst enthalten ist.

Um den pH-Wert der erfindungsgemäßen Lösung in einem pH-Wert Bereich von 2,8 bis 4,8 möglichst konstant zu halten und damit einhergehend eine erhöhte Stabilität der erfindungsgemäßen Lösung dauerhaft zu gewährleisten, ist es entsprechend einer weiter bevorzugten Ausführungsform der erfindungsgemäßen Lösung vorgesehen, dass in der Lösung ein Milchsäure/Lactat-Puffersystem gelöst enthalten ist.

Gemäß einer weiter bevorzugten Ausführungsform der erfindungsgemäßen Lösung ist es vorgesehen, dass die Lösung zur parenteralen Verabreichung geeignet ist, vorzugsweise zur intravenösen und/oder subkutanen Verabreichung.

Nach einer weiter bevorzugten Ausführungsform der erfindungsgemäßen Lösung ist es vorgesehen, dass die Lösung frei von Stabilisatoren ist, insbesondere frei von Chelatoren und Antioxidantien. Überraschenderweise wurde festgestellt, dass die erfindungsgemäße Lösung trotz Abwesenheit von Chelatoren wie Ethylendiamintetraessigsäure (EDTA) und Antioxidantien ausreichend stabil ist.

Um die physiologische Toleranz der erfindungsgemäßen Lösung weiter zu verbessern ist es entsprechend einer weiter bevorzugten Ausführungsform der erfindungsgemäßen Lösung vorgesehen, dass die Lösung frei von einem organischen Lösungsmittel ist.

Gemäß einer weiter bevorzugten Ausführungsform der erfindungsgemäßen Lösung ist es vorgesehen, dass die Lösung frei von Liposomen und/oder frei von Detergentien ist. Diese Maßnahme trägt ebenfalls zur Verbesserung der physiologischen Toleranz der erfindungsgemäßen Lösung bei.

Nach einer weiter bevorzugten Ausführungsform der erfindungsgemäßen Lösung ist es vorgesehen, dass die Lösung steril ist.

Eine typische erfindungsgemäße Lösung enthält 65 Gew.-% bis 99,85 Gew.-% Wasser, 0,05 Gew.-% bis 1 Gew.-% Bortezomib (in freier und veresterter Form berechnet als freies Bortezomib), 0,1 bis 20 Gew.-% Mannitol (in freier und veresterter Form berechnet als freies Mannitol) sowie 0 Gew.-% bis 14 Gew.-% pharmazeutische Hilfsstoffe, beispielsweise NaCl und/oder ein organisches Lösungsmittel.

Eine weitere typische erfindungsgemäße Lösung enthält 80 Gew.-% bis 99,85 Gew.-% Wasser, 0,05 Gew.-% bis 1 Gew.-% Bortezomib (in freier und veresterter Form berechnet als freies Bortezomib), 0,1 bis 5 Gew.-% Mannitol (in freier und veresterter Form berechnet als freies Mannitol) sowie 0 Gew.-% bis 14 Gew.-% pharmazeutische Hilfsstoffe, beispielsweise NaCl und/oder ein organisches Lösungsmittel.

Die vorliegende Erfindung betrifft ferner die Verwendung der erfindungsgemäßen Lösung zur Behandlung des multiplen Myeloms und/oder des Mantelzelllymphoms.

Die vorliegende Erfindung betrifft ferner einen luftdicht verschlossenen Behälter, enthaltend die erfindungsgemäße Lösung.

Entsprechend einer bevorzugten Ausführungsform des erfindungsgemäßen Behälters ist es vorgesehen, dass der Behälter aus einem Material gebildet ist, das für Sauerstoff nicht permeabel ist.

Gemäß einer weiter bevorzugten Ausführungsform des erfindungsgemäßen Behälters ist es vorgesehen, dass der Behälter aus Glas gebildet ist.

Nach einer weiter bevorzugten Ausführungsform des erfindungsgemäßen Behälters ist es vorgesehen, dass der Behälter ein Vial ist.

Entsprechend einer bevorzugten Ausführungsform des erfindungsgemäßen Behälters ist es vorgesehen, dass der Kopfraum des Behälters mit einem Inertgas befüllt ist. Der Kopfraum des erfindungsgemäßen Behälters ist der Raum des Innenraums des erfindungsgemäßen Behälters, der nicht von der erfindungsgemäßen Lösung eingenommen wird. Als Inertgas kommen beispielsweise die Edelgase und Stickstoff in Betracht, wobei aus Kostengründen insbesondere Stickstoff bevorzugt ist.

Entsprechend einer weiter bevorzugten Ausführungsform des erfindungsgemäßen Behälters ist es vorgesehen, dass der Behälter im Innenraum, vorzugsweise unmittelbar nach dem vollständigen Verschluss, ein Sauerstoff-Bortezomib-Molverhältnis von kleiner als 1:40 aufweist, vorzugsweise ein Sauerstoff-Bortezomib-Molverhältnis von kleiner als 1:60 und besonders bevorzugt ein Sauerstoff-Bortezomib-Molverhältnis von 1:60 bis zu 1:100. Bei der Berechnung besagten Molverhältnisses wird hinsichtlich Bortezomib sowohl in der erfindungsgemäßen Lösung frei vorliegendes als auch verestertes Bortezomib berücksichtigt. Der Sauerstoffgehalt in der Lösung ist erfindungsgemäß mit einem Sauerstoff-Sensor basierend auf der Clark-Elektrode (US 2,913,386) gemäß Allen J., Lab Medicine 2003(34), 544-547)zu bestimmen. Der Sauerstoffgehalt im Kopfraum des erfindungsgemäßen Behälters ist erfindungsgemäß bevorzugt mittels Gaschromatographie unter Verwendung eines Flammenionisationsdetektors oder mittels Nahinfrarot-Dioden- Lasermessung gemäß Posset et al., Pharm Ind. 77(5), 739-747 (2015), zu bestimmen.

Gemäß einer weiter bevorzugten Ausführungsform des erfindungsgemäßen Behälters ist es vorgesehen, dass im Kopfraum des Behälters ein Druck in einem Bereich von 870 mbar bis 1085 mbar herrscht, wobei ein Druck von 950 mbar bis 1050 mbar, ein Druck von 1010 mbar bis 1050 mbar oder ein Druck von 1020 mbar bis 1030 mbar bevorzugt ist.

Entsprechend einer bevorzugten Ausführungsform des erfindungsgemäßen Behälters ist es vorgesehen, dass der Kopfraum des Behälters, vorzugsweise unmittelbar nach dem verschließen des Behälters, einen Sauerstoffgehalt von maximal 0,15 Vol.-% aufweist, vorzugsweise einen Sauerstoffgehalt von 0,15 Vol.-% bis 0,005 Vol.-%. Der Sauerstoffgehalt im Kopfraum des erfindungsgemäßen Behälters ist erfindungsgemäß bevorzugt mittels Gaschromatographie unter Verwendung eines Flammenionisationsdetektors oder mittels Nahinfrarot-Dioden-Lasermessung gemäß Posset et al., Pharm Ind. 77(5), 739-747 (2015), zu bestimmen.

Nach einer weiter bevorzugten Ausführungsform des erfindungsgemäßen Behälters ist es vorgesehen, dass das Verhältnis von Kopfraum zu Innenraum des abgedichteten Behältnisses einen Wert von kleiner als 0,95 aufweist, bevorzugt einen Wert von 0,75 bis 0,95. In einer weiteren bevorzugten Ausführungsform ist es vorgesehen, dass das Verhältnis von Kopfraum zu Innenraum des abgedichteten Behältnisses einen Wert von kleiner als 0,75 aufweist, bevorzugt einen Wert von 0,5 bis 0,75.

Die nachstehende Beschreibung einiger bevorzugter Ausführungsformen dient der Erläuterung der Erfindung.

Die erfindungsgemäße Lösung bzw. der erfindungsgemäße Behälter kann beispielsweise unter Zuhilfenahme der Offenbarung der US 6,274,169 hergestellt werden, insbesondere unter Zuhilfenahme der dort abgebildeten und beschriebenen Vorrichtungen.

### Ausführungsbeispiel 1

Herstellung einer erfindungsgemäßen gebrauchsfertigen Lösung:
1250,0 g Mannitol wurden in 48,88 kg Wasser unter Rühren gelöst. Durch die so erhaltene Lösung wurde unter Rühren solange sterilfiltrierter Stickstoff hindurch geleitet, bis der Sauerstoffgehalt der Lösung 0,5 mg/l betrug (gemessen mittels Clark-Elektrode, Mettler Toledo InPro 6800). Die so hergestellte Mannitol-Lösung wurde dann auf eine Temperatur von 45 °C erwärmt. In der erwärmten Mannitol-Lösung wurden anschließend zunächst 125,0 g Bortezomib und dann 450,0 g NaCl unter Rühren gelöst.

Die wie vorstehend ausgeführt erhaltene Bortezomib-Mannitol-Lösung wurde nach dem Lösen des NaCl's zunächst mit raumtemperiertem Wasser auf ein finales Volumen von 50 l aufgefüllt und dann auf eine Temperatur von 20 °C abgekühlt. Anschließend wurde der pH der abgekühlten Lösung mittels 1 %iger wässriger NaOH- bzw. HCl-Lösung auf einen Wert von 4,3 eingestellt. Nach der pH-Wert-Einstellung wurde durch die Bortezomib-Mannitol-Lösung unter Rühren nochmals solange sterilfiltrierter Stickstoff hindurch geleitet, bis der Sauerstoffgehalt der Lösung 0,5 mg/l betrug (gemessen mittels Clark-Elektrode, Mettler Toledo InPro 6800). Danach wurde die Bortezomib-Mannitol-Lösung mittels einer Sterilfiltrationskapsel unter Anwendung eines Stickstoffdrucks von 1,5 bar sterilfiltriert.

### Herstellung eines erfindungsgemäßen Behälters:

Die vorgenannte sterilfiltrierte Lösung wurde unter einer Stickstoffatmosphäre in mehrfach mit Stickstoff gespülte Vials (10R DIN, 13 mm Hals) mit einem Innenraumvolumen von 12,15 ml zu Portionen von 1,4 ml abgefüllt. Die befüllten offenen Vials wurden anschließend in eine Lyophilisierungskammer verbracht. Die Kammer wurde bei einer Temperatur von 20 °C auf einen Druck von 50 mbar +/- 10 mbar evakuiert und anschließend mit sterilfiltriertem Stickstoff geflutet, wobei ein Stickstoffdruck von 950 +/- 25 mbar eingestellt wurde. Besagter Evakuierungs-/Flut-Vorgang wurde anschließend noch zweimal wiederholt, wobei bei der zweiten Wiederholung beim Fluten mit Stickstoff der Stickstoffdruck in der Kammer auf einen Wert von 1013 mbar eingestellt wurde. Die Vials wurden in der Kammer unter Stickstoffatmosphäre mit einem Gummistopfen verschlossen, der Kammer entnommen und dann mit einer Aluminiumkappe verbördelt. Der Sauerstoffgehalt im Kopfraum des Vials betrug unmittelbar nach dem Verschließen und Verbördeln 0,15 Vol.-% (gemessen mittels Gaschromatographie mit Flammenionisationsdetektor: Probenzug: Verdrängung des Gases im Vialkopfraum durch eingepresstes Wasser bei gleichzeitiger Entnahme über ein Dreiwegeventil; das Vial wird hierzu *bottom up* unter Wasser getaucht in einer Fixierung eingespannt; Bestimmung von Sauerstoff neben Stickstoff; Säule: Molekularsieb 5 A, 50 m, 0,53 *µ*m; Trägergas: Helium (ca. 80 kPa); Injektion: manuell; Bemerkung: da der Sauerstoffgehalt in der Probe deutlich kleiner als die Bestimmungsgrenze (Ref-Stdd. 0,50%(V/V) Sauerstoff) ist, kann über eine Einpunktkalibrierung-Limittest am LOQ (0.1% (V/V)) ausgewertet werden).

### Stabilitätsuntersuchung

Befüllte Vials gemäß Ausführungsbeispiel 1 wurden über einen Zeitraum von 6 Monaten bei Temperaturen von 2-8 °C, 15 °C und 25 °C eingelagert. Nach einem, zwei, drei und sechs Monat/en wurde der Bortezomibgehalt der Lösungen mittels *Eigh-performance liquid chromatography* (HPLC) wie weiter unten ausgeführt bestimmt. In der nachstehenden Tabelle sind die relativen Bortezomibgehalte der eingelagerten Lösungen in Bezug auf ursprünglich eingesetztes Bortezomib in Prozent angegeben.

| Zeit | 2-8 °C | 15 °C | 25 °C |
|---|---|---|---|
| 1 Monat | - | - | 101,4 |
| 2 Monate | 101,8 | 101,5 | 100,9 |
| 3 Monate | 102,3 | 102,1 | 100,7 |
| 6 Monate | 101,1 | 100,2 | 97,9 |

### Ausführungsbeispiel 2

### (pH-Wert-Abhängigkeit der Stabiltät)

Es wurden Vials analog Ausführungsbeispiel 1 präpariert, mit dem einzigen Unterschied, dass der pH der Lösung mit 1N Salzsäure bzw. 1N Natronlauge auf einen Wert von 3,0, 3,5, 4,0, 4,5, 5,0, 5,5, 6,0 bzw. 6,5 eingestellt wurde.

### Stabilitätsuntersuchung

Befüllte Vials gemäß Ausführungsbeispiel 2 wurden über einen Zeitraum von 4 Wochen bei einer Temperatur von 40 °C eingelagert. Nach einer, zwei, drei und vier Woche/n der Einlagerung wurde der Bortezomibgehalt der Lösungen mittels HPLC wie weiter unten ausgeführt bestimmt.

Die Auswertung der Bortezomibgehalte der Lösungen ergab, dass eine Lösung die höchste Stabilität aufweist, wenn der pH-Wert der Lösung auf einen Wert von 3,5 bis 4,5 eingestellt wurde.

### HPLC

Der relative Gehalt der Lösungen an Bortezomib bezogen auf die ursprüngliche Einwaage wurde mittels HPLC gemäß Europäischem Arzneibuch, 8. Ausgabe, Ziffer 2.2.29, Seiten 60 bis 62 mit den folgenden Systemparametern bestimmt:
- Gerät: HPLC SpectraSYSTEM von Thermo Electron Corp. ausgestattet mit Diodenfelddetektor sowie einen Säulenofen;
- Säule: SymmetryShield RP 18, 5 µm, 250 x 4,6 mm, Waters Artikelnummer 186000112;
- mobile Phase A: entgaste Mischung von 715 ml Wasser, 285 ml Acetonitril und 1 ml Ameisensäure;
- mobile Phase B: entgaste Mischung von 200 ml Wasser, 800 ml Methanol und 1 ml Ameisensäure;
- Flussrate: 1,0 ml/min;
- Injektionsvolumen: 20 µl;
- Detektion: Diodenfeldspektralphotometer mit einer bei 270 nm festgelegten Wellenlänge und einer spektralen Abtastung von 200 bis 420 nm;
- Temperatur: 35 °C;
- Externer Standard: 10 mg Bortezomib gelöst in 20 ml Diluent
- Probenvorbereitung: 1 ml einer Bortezomib-Lösung mit einer Konzentration von 2,5 mg/ml werden mit Diluent auf ein Volumen von 5 ml verdünnt.
- Diluent: Wasser / Acetonitril 70:30 (Vol.-%/Vol.-%)
- Gradient:

| Zeit [min] | mobile Phase A [Vol. -%] | mobile Phase A [Vol.-%] |
|---|---|---|
| 0 - 20 | 100 | 0 |
| 20 - 35 | 100 → 0 | 0 → 100 |
| 35 - 50 | 0 | 100 |
| 50 - 52 | 0 →100 | 100 → 0 |
| 52 - 57 | 100 | 0 |

## Patentansprüche

1. Gebrauchsfertige flüssige pharmazeutische Lösung, umfassend
- ein wässriges Lösungsmittel;
- einen Bortezomibester, der in dem Lösungsmittel gelöst enthalten ist.

2. Lösung nach Anspruch 1, **dadurch gekennzeichnet, dass** die Bortezomibester-Bildungskomponente ein Polyol ist.

3. Lösung nach einem der voranstehenden Ansprüche, **dadurch gekennzeichnet, dass** die Bortezomibester-Bildungskomponente ein Alditol ist.

4. Lösung nach einem der voranstehenden Ansprüche, **dadurch gekennzeichnet, dass** die Bortezomibester-Bildungskomponente Mannitol ist.

5. Lösung nach einem der voranstehenden Ansprüche, **dadurch gekennzeichnet, dass** die Bortezomibester-Bildungskomponente in einem zumindest 10-fachen molaren Überschuss in der Lösung gelöst enthalten ist in Bezug auf in der Lösung enthaltenes Bortezomib, bevorzugt in einem 10 bis 400-fachen molaren Überschuss, besonders bevorzugt in einem 10 bis 100-fachen molaren Überschuss und ganz besonders bevorzugt in einem 15 bis 30-fachen molaren Überschuss.

6. Lösung nach einem der voranstehenden Ansprüche, **dadurch gekennzeichnet, dass** der Gehalt der Lösung an Bortezomibester bezogen auf Bortezomib 0,5 mg/ml bis 10 mg/ml beträgt, bevorzugt 1,0 mg/ml bis 5 mg/ml und besonders bevorzugt 2,5 mg/ml.

7. Lösung nach einem der voranstehenden Ansprüche, **dadurch gekennzeichnet, dass** in der Lösung ferner NaCl gelöst enthalten ist.

8. Lösung nach einem der voranstehenden Ansprüche, **dadurch gekennzeichnet, dass** die Lösung einen pH-Wert in einem Bereich von 3 bis 6 aufweist, bevorzugt in einem Bereich von 3 bis 5 und besonders bevorzugt in einem Bereich von 3,5 bis 4,5.

9. Lösung nach einem der voranstehenden Ansprüche, **dadurch gekennzeichnet, dass** in der Lösung ein Essigsäure/Acetat-Puffersystem gelöst enthalten ist.

10. Lösung nach einem der voranstehenden Ansprüche, **dadurch gekennzeichnet, dass** die Lösung zur parenteralen Verabreichung geeignet ist, vorzugsweise zur intravenösen und/oder subkutanen Verabreichung.

11. Lösung nach einem der voranstehenden Ansprüche, **dadurch gekennzeichnet, dass** die Lösung frei von einem organischen Lösungsmittel ist.

12. Luftdicht verschlossener Behälter, enthaltend eine Lösung nach einem der voranstehenden Ansprüche.

13. Behälter nach Anspruch 12, **dadurch gekennzeichnet, dass** der Behälter ein Vial ist.

14. Behälter nach einem der voranstehenden Ansprüche, **dadurch gekennzeichnet, dass** der Kopfraum des Behälters mit einem Inertgas befüllt ist.

15. Behälter nach einem der voranstehenden Ansprüche, **dadurch gekennzeichnet, dass** der Behälter im Innenraum ein Sauerstoff-Bortezomib-Molverhältnis von kleiner als 1:40 aufweist, vorzugsweise ein Sauerstoff-Bortezomib-Molverhältnis von kleiner als 1:60.
